# EUROPÄISCHE PATENTANMELDUNG

(11) **EP 0 529 469 A2**
(43) Veröffentlichungstag der Anmeldung: **03.03.1993**
(21) Anmeldenummer: 92114033.1
(22) Anmeldetag: 18.08.1992
(51) Int. Cl.: C07C 271/22, C07C 229/26, C07D 241/08

(54) **Neue substituierte 2,3-Diaminosäuren, Verfahren zu ihrer Herstellung und ihre Verwendng als Zwischenstufen für peptidische Wirkstoffe**

(30) Priorität: 30.08.1991 DE 4128790
(71) Anmelder: BAYER AG, 51368 Leverkusen (DE)
(72) Erfinder: Mittendorf, Joachim, Dr., W-5600 Wuppertal (DE); Hartwig, Wolfgang, Dr., W-5600 Wuppertal (DE)

(57) **Zusammenfassung**

Neue substituierte 2,3-Diaminosäuren, Verfahren zu ihrer enantioselektiven Herstellung und ihre Verwendung als Zwischenstufen für die Synthese peptidischer Wirkstoffe, insbesondere für Renin-Inhibitoren und HIV-Protease-Inhibitoren.

## Beschreibung

Die Erfindung betrifft neue substituierte 2,3-Diaminosäuren, Verfahren zu ihrer enantioselektiven Herstellung und ihre Verwendung als Zwischenstufen für die Synthese peptidischer Wirkstoffe, insbesondere für Renin-Inhibitoren und HIV-Protease-Inhibitoren.

Es sind bereits Diaminosäuren bekannt, vgl. DE-OS 3 810 973 oder JAP-Appl. 52 021 327 oder Rapoport et al. J. Org. Chem. (1990) 5017 ff., die sich jedoch in ihrer chemischen Struktur eindeutig von den erfindungsgemäßen 2,3-Diaminosäuren unterscheiden.

Die Erfindung betrifft neue substituierte 2,3-Diaminosäuren der allgemeinen Formel (I) 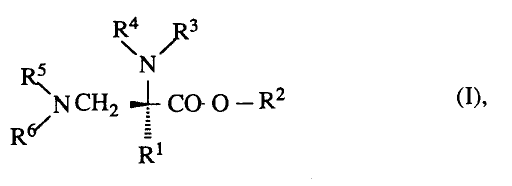
in welcher
- R¹: für Alkyl mit 1 bis 10 C-Atomen, Alkenyl mit bis zu 4 C-Atomen, Phenyl, Benzyl oder Phenethyl steht,
- R²: für Wasserstoff, Benzyl oder Alkyl mit 1 bis 6 C-Atomen steht,
- R³ und R⁴: gleich oder verschieden sind und jeweils für Wasserstoff oder eine Schutzgruppe wie BOC (tert.-Butyloxycarbonyl), Acetyl, Benzoyl, Benzyloxycarbonyl, Fluorenyl-methyloxycarbonyl, Formyl, Trifluoracetyl, 2,2,2-Trichlorethylcarbamat, 2-Haloethylcarbamat, 2-Trimethylsilylcarbamat stehen und
- R⁵ und R⁶: gleich oder verschieden sind und jeweils die für R³ und R⁴ angegebene Bedeutung haben,
sowie deren Salze.

Von besonderem Interesse sind Verbindungen der allgemeinen Formel (I), in welcher
- R¹: für Alkyl oder Alkenyl mit bis zu 4 C-Atomen, Benzyl oder Phenyl steht und
- R² bis R⁶: die oben angegebene Bedeutung haben.

Die Herstellung der erfindungsgemäßen Verbindungen in den jeweils enantiomeren Formen erfolgt, indem man enantiomerenreine Azidoverbindungen der allgemeinen Formel (II) 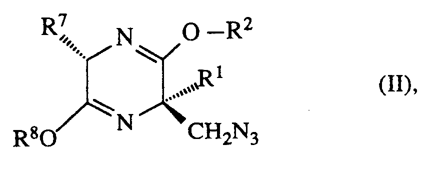
in welcher
- R¹ und R²: die oben angegebene Bedeutung haben,
- R⁷: für Methyl, iso-Propyl oder tert.-Butyl steht, und
- R⁸: die für R¹ angegebene Bedeutung hat und gleich oder verschieden von R¹ ist,
nach üblichen Methoden zu den entsprechenden Aminoverbindungen reduziert, diese Aminogruppen mit einer üblichen Schutzgruppe blockiert und anschließend die Bislactimetherringe aufspaltet und gegebenenfalls die entstandene primäre Aminogruppe (R³ und R⁴ = H) durch übliche Schutzgruppen substituiert.

Die als Ausgangsverbindungen eingesetzten Bislactimetherhalogenide sind bekannt oder können nach bekannten Methoden hergestellt werden (vgl. DE-OS 2 934 252).

Das erfindungsgemäße Verfahren kann beispielhaft durch folgendes Reaktionsschema erläutert werden:

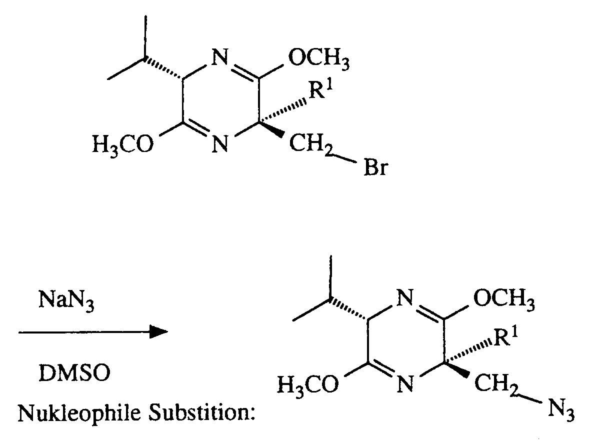

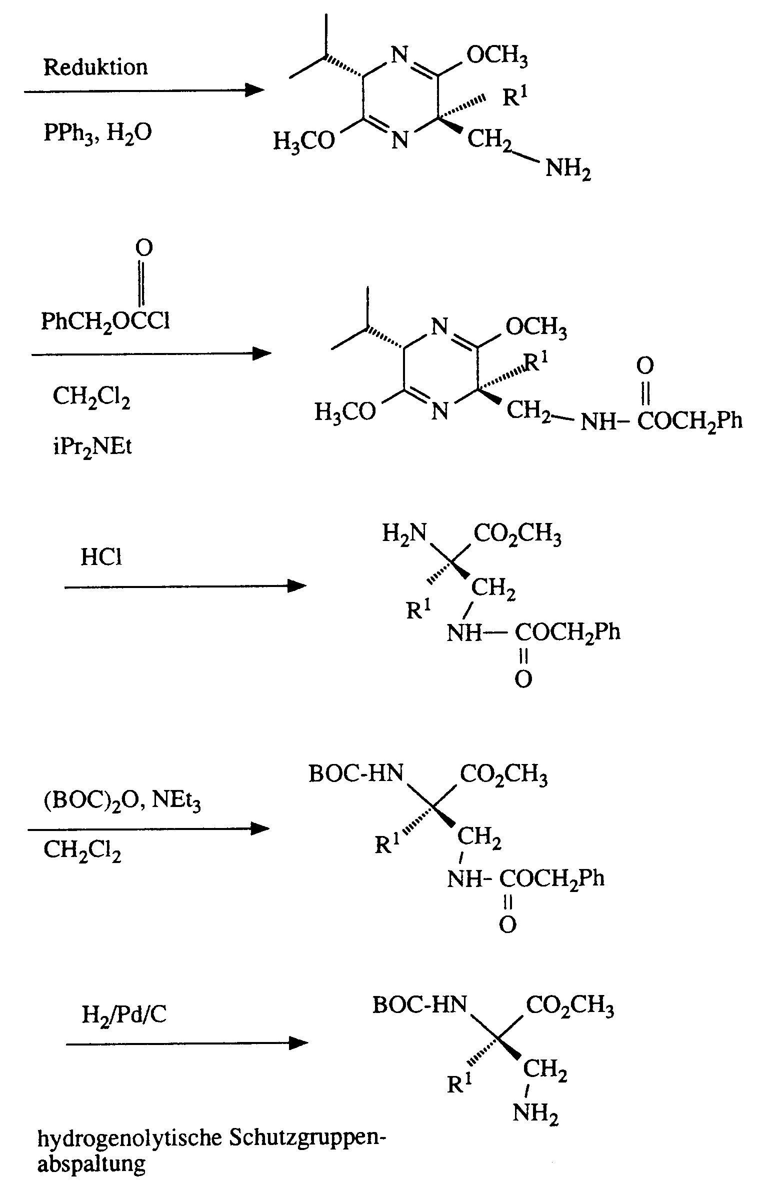

Durch die Möglichkeit, die erfindungsgemäßen 2,3-Diaminosäuren der allgemeinen Formel (I) durch differenzierbare Aminoschutzgruppen zu substituieren, ist es möglich, sie wahlweise über die 2- oder die 3-Aminofunktion in peptidische Wirkstoffe einzubauen. Sie ermöglichen somit dem Fachmann in einfacher Weise die Synthese neuer peptidischer Wirkstoffe.

Gegenstand der Erfindung sind auch die Zwischenprodukte der allgemeinen Formel (II) 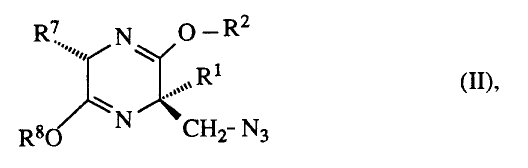
in welcher
R¹, R², R⁷ und R⁸ die oben angegebene Bedeutung haben,
sowie deren Verwendung zur Herstellung und Verbindung der allgemeinen Formel (I).

Die zur Herstellung von Verbindungen der allgemeinen Formel (II) einsetzbaren Halogen- bzw. Bromverbindungen sind bekannt oder können nach bekannten Methoden hergestellt werden (vgl.: Synthesis 1983, 37 und Angew. Chem. 1989 101,633).

Die nachfolgenden Ausführungsbeispiele erläutern den Erfindungsgegenstand. Die Beispiele 1 und 2 stützen die Herstellung des Zwischenproduktes der allgemeinen Formel (II). Die Beispiele Nr. 3 bis 6 erläutern der Reduktion der Azidogruppe zu entsprechenden Aminoverbindungen, die teilweise schon durch Schutzgruppen substituiert sind. Die Beispiele 7 bis 10 erläutern die Aufspaltung des Ringes zu den erfindungsgemäßen Verbindungen der Formel (I).

### Ausführungsbeispiele

### Experimentelle Beispiele

Die Herstellung der Ausgangsverbindung 1 erfolgte nach Ref. 2 und 5 der Publikation (Synthesis 1983, 37; Angew. Chem. 1989, 101, 633).

### Beispiel 1

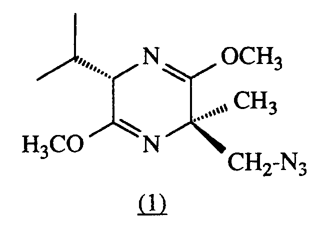

Eine Lösung von 3,70 g (12,7 mmol) der Verbindung 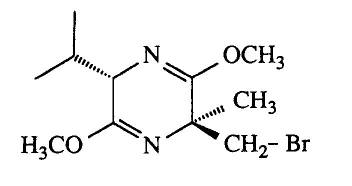
in 35 ml DMSO wird mit 3,39 g (52,1 mmol) Natriumazid versetzt und 48 h auf 70°C erhitzt. Nach Zugabe von 50 ml Wasser wird dreimal mit je 50 ml Petrolether extrahiert und die vereinigten organischen Phasen werden über Natriumsulfat getrocknet, filtriert und im Vakuum eingeengt. Der Rückstand wird an Kieselgel chromatographiert.
Ausbeute: 3,22 g (100 %)
C₁₁H₁₉N₅O₂ (253.3)
R_{F} = 0.58 (Ether: Petrolether = 1:5)

### Beispiel 2

Analog Beispiel 1 wurde hergestellt: 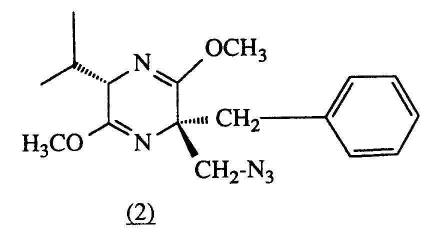
Ausbeute: 100 %
C₁₇H₂₃N₅O₂ (329.3)
R_{F} = 0.35 (Toluol)

### Beispiel 3

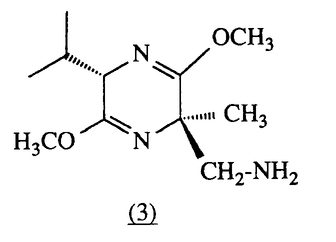

Eine Lösung von 0,25 g (1,0 mmol) (1), 0,26 g (1,0 mmol) Triphenylphosphin und 0,027 g (1,5 mmol) H₂O in 10 ml THF wird 48 h gerührt. Das Lösungsmittel wird im Vakuum abgezogen und der Rückstand in 5 ml Ether/Petrolether (1:1) aufgenommen. Triphenylphosphinoxid wird abfiltriert, das Lösungsmittel im Vakuum abgezogen und der Rückstand an Kieselgel chromatographiert (Ether:Acetonitril:konz. wäßrige NH₃-Lösung = 10:1:0.1)
Ausbeute: 0,17 g (75%)
C₁₁H₂₁N₃O₂ (227.3)
R_{F}: 0.26 (Ether:AcCN:konz. NH₃ = 10:1:0.1)

### Beispiel 4

Analog Beispiel 3 wurde aus (2) hergestellt: 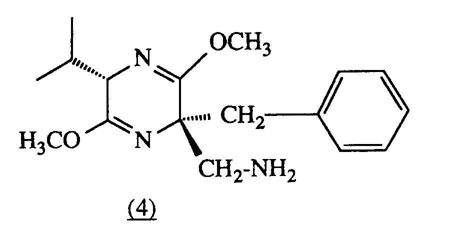
Ausbeute: 55 %
C₁₇H₂₅N₃O₂ (303.4)
R_{F}: 0.31 (Ether:AcCN:konz. NH₃ = 10:1:0.1)

### Beispiel 5

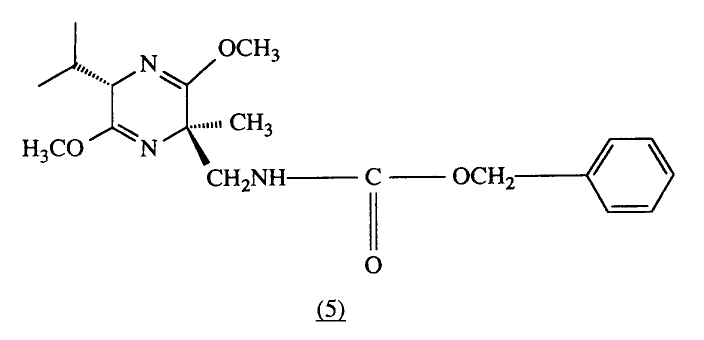

Zur Lösung von 0,23 g (1,0 mmol) (3) und 0,39 g (3,0 mmol) Ethyldiisopropylamin in 5 ml Dichlormethan werden 0,17 g (1,0 mmol) Benzylchloroformiat getropft. Es wird 3 h bei Raumtemperatur gerührt, anschließend mit Wasser, 1 % Salzsäure und ges. wäßrige NaHCO₃-Lösung gewaschen und über Na₂SO₄ getrocknet. Das Lösungsmittel wird im Vakuum abgezogen und der Rückstand an Kieselgel chromatographiert (Ether:Petrolether = 1:1).
Ausbeute: 0.31 g (86 %)
C₁₉H₂₇N₃O₄ (361.4)
R_{F} = 0.49 (Ether:Petrolether = 1:1)

### Beispiel 6

Analog Beispiel 5 wurde hergestellt: 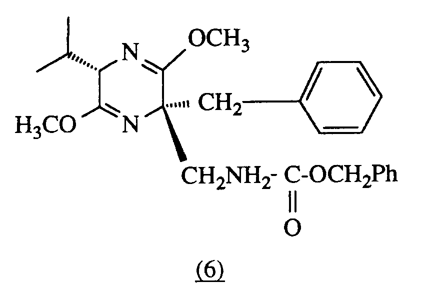
Ausbeute: 75 %
C₂₅H₃₁N₃O₄ (437.5)
Smp.: 70°C

### Beispiel 7

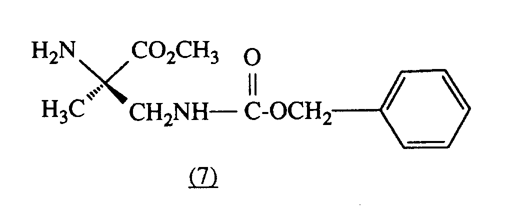

Eine Lösung von 4,10 g (11,3 mmol) (5) in 227 ml (22,7 mmol) 0,1 N HCl und 20 ml Acetonitril wird 5 h bei Raumtemperatur gerührt. Das Acetonitril wird im Vakuum abgezogen, die wäßrige Phase wird 2x mit je 50 ml Ether extrahiert, die Etherphasen werden verworfen und die wäßrige Phase auf ein Volumen von ca. 30 ml im Vakuum eingeengt. Man überschichtet mit 50 ml Ether und versetzt mit konz. NH₃-Lösung bis pH 9-10. Die Phasen werden getrennt und die wäßrige Phase wird noch 2x mit je 50 ml Ether extrahiert. Die vereinten Etherphasen werden über Na₂SO₄ getrocknet, filtriert und das Solvens wird im Vakuum abgezogen. Valinmethylester wird am Kugelrohr bei 0,1 Torr/60°C abdestilliert und der Rückstand wird an Kieselgel (Ether:Acetonitril:konz. NH₃= 10:1:0.1) chromatographiert.
Ausbeute: 1,78 g (59%)
C₁₃H₁₈N₂O₄ (266.3)
R_{F}: 0.33 (Ether:Acetonitril:konz. NH₃ = 10:1:0.1)

### Beispiel 8

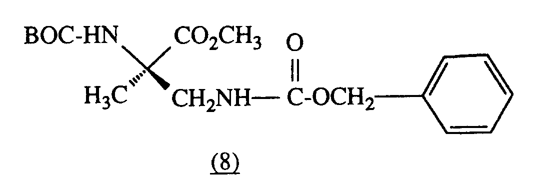

Eine Lösung von 1,78 g (6,68 mmol) (7) und 2,29 g (22,6 mmol) Triethylamin in 50 ml Dictuormethan wird mit 3,70 g (16,95 mmol) Di-tert.-butyl-dicarbonat versetzt und 12 h bei Raumtemperatur gerührt.

Die Lösung wird zweimal mit je 50 ml H₂O gewaschen, über Na₂SO₄ getrocknet und im Vakuum eingeengt. Der Rückstand wird an Kieselgel chromatographiert (Toluol:Essigester = 3:1).
Ausbeute: 2,20 g (90 %)
C₁₈H₂₆N₂O₆ (366.41)
R_{F}: 0.41 (Toluol:Essigester = 3:1)

### Beispiel 9

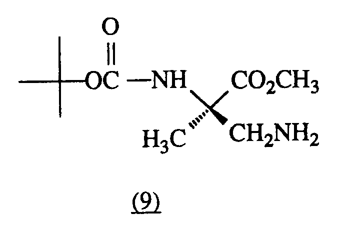

Eine Lösung von 1,74 g (4,91 mmol) (8) in 70 ml Ethanol wird mit 100 mg Palladium (10 % auf Aktivkohle) versetzt und 8 h bei Raumtemperatur und 3 bar mit H₂ hydriert. Die Lösung wird filtriert und im Vakuum eingeengt. Der Rückstand wird an Kieselgel chromatographiert (Ether:Acetonitril:konz. NH₃ = 10:1:0.1).
Ausbeute: 1,00 g (87 %)
C₁₀H₂₀N₂O₄ (232.80)
R_{F}: 0.24 (Ether:Acetonitril:konz. NH₃ = 10:1:0.1)

### Beispiel 10

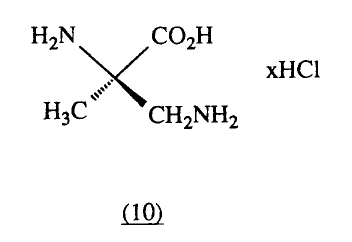

Eine Lösung von 0,440 g (1,89 mmol) (9) in 10 ml 3 N HCl wird 3 h unter Rückfluß erhitzt. Die Lösung wird im Vakuum eingeengt, der Rückstand 3 h bei 0,1 Torr/60°C getrocknet und anschließend mit 7 ml Ethanol und 1,4 ml Propenoxid versetzt. Die Lösung wird 3 h unter Rückfluß erhitzt. Man läßt auf Raumtemperatur abkühlen, ausgefallenes Produkt wird abgesaugt und mit Ethanol und Ether gewaschen.
Ausbeute: 0,220 g (75 %)
C₄H₁₀N₂O₂ x HCl (118.1x36.5)
α_{D}²⁰ = -3.47 (c = 0.7, H₂O)

## Patentansprüche

1. 2,3-Diaminosäuren der allgemeinen Formel (I) 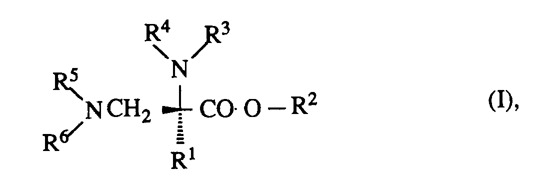 in welcher
R¹ für Alkyl mit 1 bis 10 C-Atomen, Alkenyl mit bis zu 4 C-Atomen, Phenyl, Benzyl oder Phenethyl steht,
R² für Wasserstoff, Benzyl oder Alkyl mit 1 bis 6 C-Atomen steht,
R³ und R⁴ gleich oder verschieden sind und jeweils für Wasserstoff oder eine Schutzgruppe wie BOC (tert.-Butyloxycarbonyl), Acetyl, Benzoyl, Benzyloxycarbonyl, Fluorenyl-methyloxycarbonyl, Formyl, Trifluoracetyl, 2,2,2-Trichlorethylcarbamat, 2-Haloethylcarbamat, 2-Trimethylsilylcarbamat stehen und
R⁵ und R⁶ gleich oder verschieden sind und jeweils die für R³ und R⁴ angegebene Bedeutung haben,
sowie deren Salze.

2. Verbindungen der allgemeinen Formel (I) gemäß Anspruch 1, in welcher
R¹ für Alkyl oder Alkenyl mit bis zu 4 C-Atomen, Benzyl oder Phenyl steht und
R² bis R⁶ die in Anspruch 1 angegebene Bedeutung haben.

3. Verfahren zur Herstellung von Verbindungen der allgemeinen Formel (I) gemäß Anspruch 1, dadurch gekennzeichnet, daß man enantiomerenreine Azidoverbindungen der allgemeinen Formel (II) 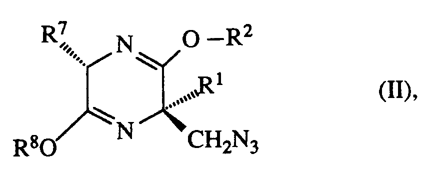 in welcher
R¹ und R² die oben angegebene Bedeutung haben,
R⁷ für Methyl, iso-Propyl oder tert.-Butyl steht, und
R⁸ die für R¹ angegebene Bedeutung hat und gleich oder verschieden von R¹ ist,
nach üblichen Methoden zu den entsprechenden Aminoverbindungen reduziert, diese Aminogruppen mit einer üblichen Schutzgruppe blockiert und anschließend die Bislactimetherringe aufspaltet und gegebenenfalls die entstandene primäre Aminogruppe (R³ und R⁴ = H) durch übliche Schutzgruppen substituiert.

4. Verbindungen der allgemeinen Formel (II) 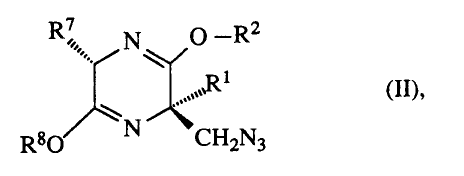 in welcher
R¹ und R² die im Anspruch 1 angegebene Bedeutung haben,
R⁷ für Methyl, iso-Propyl oder tert.-Butyl steht, und
R⁸ die für R¹ angegebene Bedeutung hat und gleich oder verschieden von R¹ ist.

5. Verwendung von Verbindungen der allgemeinen Formeln (I) und (II) als Zwischenstufen bei der Herstellung peptidischer Wirkstoffe.
